# FASCICULE DE BREVET EUROPEEN

(11) **EP 1 545 666 B1**
(45) Date de publication et mention de la délivrance du brevet: **28.09.2011**
(21) Numéro de dépôt: 03775464.5
(22) Date de dépôt: 03.10.2003
(51) Int. Cl.: A61M 5/32

(54) **CANULE ANTI-PIQUE A USAGE MEDICAL**
NADEL ZUM MEDIZINISCHEN GEBRAUCH MIT SCHUTZ GEGEN UNBEABSICHTIGTE NADELSTICHE
NON-PIERCING CANNULA FOR MEDICAL USE

(30) Priorité: 04.10.2002 FR 0212336
(43) Date de publication de la demande: 29.06.2005
(73) Titulaire: Vygon, F-95440 Ecouen (FR)
(72) Inventeur: Costello, Alan, Gloucester GL4 4WY,GB (GB)
(74) Mandataire: Texier, Christian
(86) Numéro de dépôt international: PCT/FR2003/002905
(87) Numéro de publication internationale: WO 2004/030731

(56) Documents cités:
- CH-A- 114 006
- DE-A- 19 512 607
- GB-A- 2 272 895
- US-A- 2 748 769
- US-A- 4 537 593
- US-A- 4 711 250
- US-A- 5 032 117

## Description

L'invention concerne une canule anti-pique à usage médical pour aspirer le contenu d'un conteneur ou le remplir, à travers un bouchon non préfendu.

Le conteneur est par exemple un flacon, une ampoule ou une poche.

Habituellement pour aspirer des solutions contenues dans des bouteilles, ou poches, ou ampoules ou pour mélanger ces solutions avec des médicaments en poudre, il faut percer les bouchons en élastomère équipant ces récipients. Ces bouchons sont fréquemment en polyisoprène.

L'habitude consiste à utiliser une aiguille hypodermique, montée sur une seringue. Avec le biseau tranchant de celle-ci, on perce le bouchon, puis on aspire le contenu avec la seringue.

De cette manière, des médicaments liquides peuvent être aspirés d'une ampoule et mélangés à une solution saline par exemple, contenue dans une poche de perfusion. De même, la solution saline peut être aspirée de la poche ou bouteille et poussée dans un flacon contenant les médicaments en poudre pour le mélange (flacon appelé Vial).

Toutes ces manipulations amènent à monter une aiguille sur une seringue, à la démonter de cette seringue si on connecte le luer mâle de celle-ci dans un luer femelle, à piquer une ou plusieurs fois. D'où un risque pour l'utilisateur de se piquer.

Le passage répété du biseau de l'aiguille à travers des bouchons élastomères abîme celui-ci. On peut aussi se servir de cette aiguille pour piquer le patient ensuite en cas d'injection directe en intraveineuse ou intramusculaire, d'où une mauvaise qualité de pique. De plus, le biseau d'une aiguille hypodermique peut réaliser un carottage sur le bouchon en perçant, une particule peut entrer dans celle-ci.

L'utilisateur pouvant se piquer et l'aiguille pouvant servir à piquer le patient, il y a des risques de contaminations croisées (maladies nosocomiales).

D'autre part, l'aiguille est jetée ensuite après utilisation et peut provoquer un risque pour les personnes chargées de les évacuer ou les détruire.

L'idée d'éliminer l'aiguille biseautée tranchante n'est pas nouvelle.

Il existe sur le marché des canules en matière plastique avec extrémité ouverte tubulaire émoussée ou des canules pointues en matière plastique dure fermée à l'extrémité et munie d'un trou latéral, ou encore des canules tubulaires émoussées en métal.

Les canules tubulaires émoussées nécessitent que les bouchons soient préfendus, ce qui n'est pas toujours possible, est délicat pour la conservation dans le temps et les tenues sous-pression, et coûteux. Le plus souvent les bouchons préfendus sont en fait des valves anti-retour.

Les canules pointues en matière plastique nécessitent de gros efforts pour percer les bouchons; leur utilisation est délicate et difficile. La pointe du plastique peut casser.

Le diamètre d'une canule en matière plastique est plus grand que celui d'une canule métallique, ce qui contribue à augmenter les efforts de pénétration. La longueur utile est limitée elle aussi, compte tenu de la technologie d'injection plastique (difficultés à réaliser des broches dans les moules de petits diamètres, très rigides pour résister aux pressions d'injection). La pointe peut malgré tout faire une écorchure sur la peau de l'utilisateur, ce qui peut être dangereux en manipulant des médicaments cytotoxiques.

On connaît également (EP 1 007 114) un système constitué d'un embout de perçage formé d'une poignée en matière, plastique et d'un tube en inox coupé en bout en biais en réalisant un léger biseau. Cet embout sert à percer les bouchons assez facilement et crée une fente, il ne constitue pas une canule et le système comprend donc également une canule tubulaire émoussée en matière plastique munie d'une embase qui peut être introduite à travers le bouchon pour l'aspiration.

Le système est onéreux et nécessite deux manipulations : il faut d'abord percer avec le premier embout puis introduire la canule plastique. Par contre, il n'y a pas de risque de se piquer pour l'utilisateur, l'embout de perçage étant atraumatique.

On connaît également des canules pour prélever ou introduire un liquide à travers un bouchon non préfendu, constituées par un tube creux cylindrique qui présente une extrémité proximale ouverte munie d'un embase tubulaire de raccordement, qui présente une extrémité distale fermée pour perforer le bouchon et qui présente entre ces extrémités au moins un oeil latéral communiquant avec l'intérieur du tube (brevets US 4 537 593 et 5 032 117). Le préambule de la revendication 1 a été defini par rapport au document US 4 537 593. Ces canules à tube métallique présentent une extrémité perforante taillée en lame de rasoir et par conséquent susceptible d'être traumatisante.

La présente invention vise à fournir une canule à tube métallique pourvue d'au moins un oeil latéral et non traumatisante.

On y parvient selon l'invention avec une canule dont le tube est métallique et présente une extrémité distale aplatie de façon à déterminer deux parois de tube opposées qui se rejoignent progressivement jusqu'à former une arête atraumatique .

Dans des modes de réalisations préférés, cette canule distale présente encore une ou plusieurs des caractéristiques suivantes :
- l'arête de perforation coupe en biais l'axe du tube et présente une section droite de forme triangulaire.
- Le tube comporte au moins un oeil qui aboutit
- sensiblement à la zone de départ de l'aplatissement.
- Ledit oeil est situé dans le prolongement de l'une des dites parois opposées.
- Ledit oeil est situé sur un côté du tube à 90°des dites parois opposées.
- Lesdites parois opposées sont incurvées l'une vers l'autre et se terminent éventuellement par deux parties de paroi parallèles qui aboutissent à ladite arête.
- L'arête de perforation est plate et perpendiculaire à l'axe du tube.

On décrira ci-après des réalisations de canules conformes à l'invention en référence aux figures du dessin joint sur lequel :
- la figure 1 est une perspective d'une première réalisation de canule ;
- la figure 2 est une perspective agrandie de l'extrémité distale de la canule de la figure 1 ;
- la figure 3 est une élévation de cette extrémité vue d'un côté du tube qui ne comporte pas d'oeil ;
- la figure 4 est une élévation de cette extrémité vue d'un côté du tube qui comporte un oeil ;
- la figure 5 est une vue de cette extrémité dans un plan transversal par rapport aux parois opposées provenant de l'aplatissement de cette extrémité ;
- la figure 6 est une perspective d'une deuxième réalisation de la canule ;
- la figure 7 est une perspective agrandie de l'extrémité distale de la canule de la figure 6 ;
- la figure 8 est une élévation de cette extrémité vue du côté qui comporte un oeil ;
- la figure 9 est une élévation de cette extrémité qui montre en plan l'une des parois opposées créés par l'aplatissement du tube ;
- la figure 10 est une vue de l'extrémité de la deuxième réalisation dans un plan transversal par rapport aux parois opposées provenant de l'aplatissement de cette extrémité ;
- la figure 11 est un schéma d'une variante de réalisation, le plan de vue étant parallèle à l'ouverture de l'oeil ;
- la figure 12 est un schéma de la variante de la figure 11 dans un plan perpendiculaire à celui de la figure 11, et
- la figure 13 est une vue en long de la canule des figures 11 et 12 dans son étui de conditionnement.

Sur les figures :
- (1) désigne le tube métallique de la canule. Son diamètre est de préférence de 1, 00 millimètre.
- (2) désigne l'embase en résine de synthèse qui est moulée sur l'extrémité proximale du tube. Cette embase, de façon en soi connue, est conçue pour le raccordement de la canule par exemple à une seringue, éventuellement avec vissage.
- (3) désigne l'extrémité distale du tube de la canule.

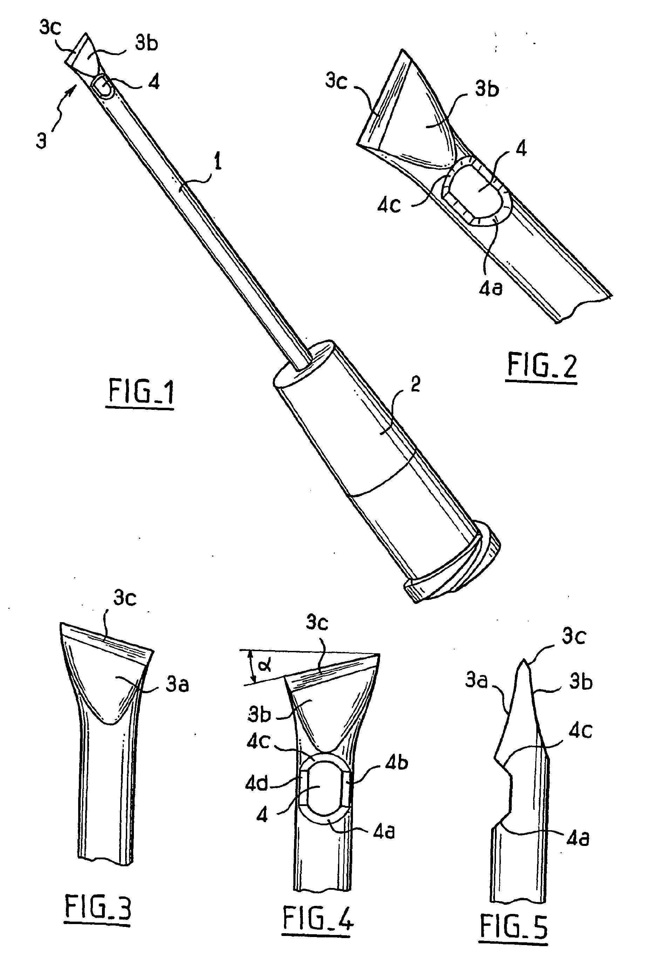

A l'extrémité distale, le tube est aplati formant deux parois opposées (3a, 3b) qui partent de la dernière section cylindrique non aplatie du tube, pour se rejoindre sur une arête (3c) qui, dans les réalisations des figures 1 à 10 est de section droite triangulaire et coupe en biais l'axe du tube.

De préférence, les deux parois opposées provenant de l'aplatissement (3a, 3b) ne sont pas planes mais sont creusées avec un rayon de courbure de 5,2 millimètres.

De préférence l'arête (3c) fait un angle α de 15° par rapport à un plan perpendiculaire à l'axe du tube et la section droite triangulaire de l'arête a un angle au sommet de 60°.

Dans la réalisation des figures là 5, le tube (1) présente un oeil latéral (4) allongé qui aboutit sensiblement à la naissance de l'une (3b) des parois formés par l'aplatissement du tube (figure 4).

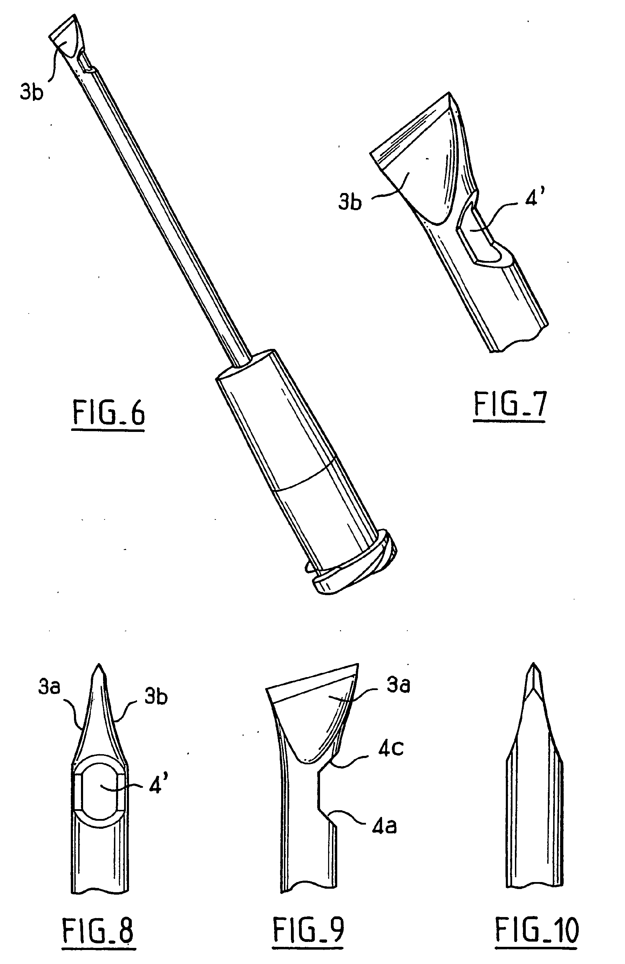

Dans la réalisation des figures 6 à 10, le tube (1) présente un oeil latéral (4') situé à 90° des deux parois (3a, 3b) formées par l'aplatissement du tube.

De préférence, l'oeil est déterminé par une ouverture formée dans la paroi du tube, bordée par des tranches (4a, 4b, 4c, 4d) qui comprennent deux tranches respectivement proximale (4a) et distale (4c) biseautée à 90° (figures 5 et 9).

Dans les réalisations préférées qui ont été représentées, l'oeil se trouve à 2 millimètres (figure 4) ou à 1, 8 millimètres (figure 8) de l'extrémité de la canule. De préférence, il s'étend sur 1,20 millimètres.

Les dimensions indiquées ci-dessus sont nominales et ont été définies avec une tolérance de ± 0,1 millimètre pour les longueurs et ± 2° pour les angles.

L'invention n'est pas limitée à ces réalisations.

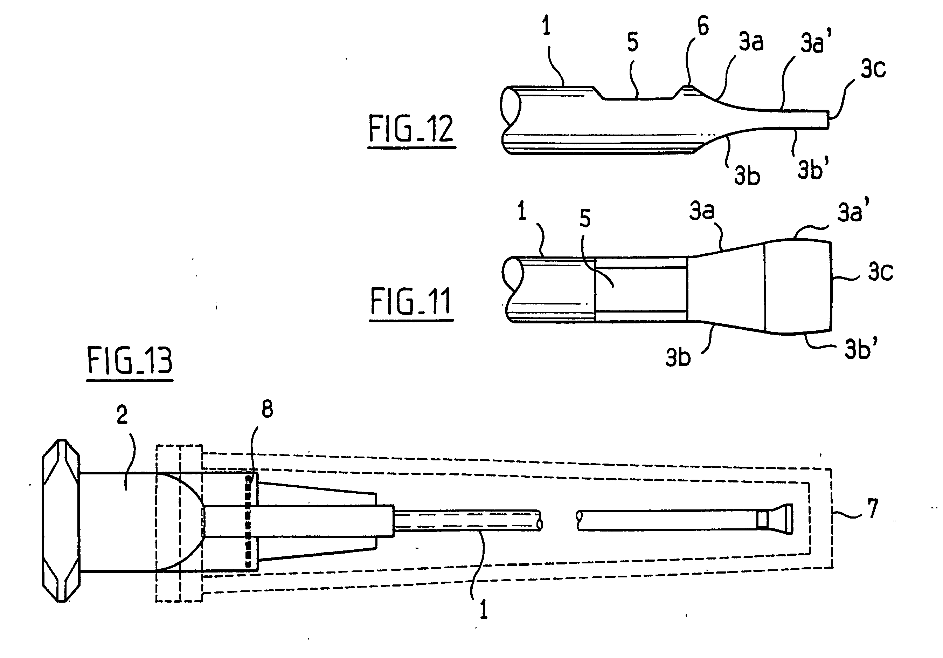

Sur les figures 11 à 13, on a représenté encore une variante de réalisation dans laquelle l'arête perforante (3c) est plate et perpendiculaire à l'axe du tube.

De préférence, comme dans l'exemple représenté, les parois (3a,3b) qui se rapprochent pour constituer ladite arête se terminent par deux parties de paroi (3a' ;3b') qui sont parallèles et aboutissent à l'arête plate.

Dans cette réalisation, le tube comporte, un oeil (5) situé à proximité de la naissance de l'aplatissement qui détermine les deux parois (3a,3b).

Dans cet exemple, cet oeil est situé dans le prolongement de l'une de ces parois.

Dans la réalisation préférée qui est représentée, les dimensions nominales sont avantageusement les suivantes :
A : 1,05 mm (tolérance +0,02 -0,02 mm)
B : 0,75 mm (tolérance +0,02 -0,02 mm)
C : 1,50 mm (tolérance +0,25 -0,25 mm)
D : 0,70 mm (tolérance +0,20 -0,05 mm)
F : 0,18 mm (tolérance +0,05 -0,18 mm)
G : 0,40 mm (tolérance +0,05 -0,40 mm)

La dimension E est par exemple d'environ 0,70 mm.

La figure 13 montre la canule dans un emballage (7).L'embase de la canule est munie d'un filtre (8).

L'invention n'est pas limitée à cet exemple.

## Revendications

1. Canule à usage médical pour prélever ou introduire un liquide à travers un bouchon non préfendu, constituée par un tube métallique creux et cylindrique (1) qui présente une extrémité proximale ouverte munie d'un embase tubulaire de raccordement (2), qui présente une extrémité distale fermée (3) par une arête (3c) pour perforer le bouchon et qui présente entre ces extrémités au moins un oeil latéral (4 ; 4') communiquant avec l'intérieur du tube, **caractérisée en ce que** l'extrémité distale du tube comporte une arête (3c) droite formée par deux parois de tube opposées (3a ;3b) qui se rejoignent progressivement et déterminées par aplatissement de l'extremité distale du tube.

2. Canule selon la revendication 1, dont l'arête (3c) présente une section droite de forme triangulaire.

3. Canule selon la revendication 2 dont la section droite de l'arête (3c) a un angle au sommet de 60°, à la tolérance près.

4. Canule selon l'une des revendications 1 à 3 dont l'arête (3c) coupe en biais l'axe du tube.

5. Canule selon la revendication 4 dont l'arête (3c) fait un angle nominal de 75° avec l'axe du tube.

6. Canule selon l'une des revendications précédentes dont le tube présente un oeil (4') situé à 90° des dites parois opposées, à la tolérance près.

7. Canule selon l'une des revendications 1 à 5 dont lesdites parois opposées (3a ; 3b) sont incurvées l'une vers l'autre.

8. Canule selon la revendication 7 dont lesdites parois opposées (3a ; 3b) sont incurvées avec un rayon de courbure de 5,2millimètres, à la tolérance près.

9. Canule selon l'une des revendications 1 à 6 dont le tube présente un oeil (4 ; 4') dont la distance nominale à l'extrémité de ladite arête (3c) est au plus égale à 2 millimètres.

10. Canule selon l'une des revendications 1 à 7 dont l'oeil (4 ; 4') s'étend parallèlement à l'axe de la canule sur 1,2 millimètres, à la tolérance prés.

11. Canule selon l'une des revendications 1 à 8 dont le tube présente un oeil (4 ; 4') constitué par une ouverture formée dans la paroi du tube et bordée par des tranches de cette paroi qui comprennent deux tranches opposées (4a ; 4c) biseautées, respectivement proximale et distale.

12. Canule selon la revendication 9 dont ledit biseau des tranches biseautées forme un angle de 90°, à la tolérance près.

13. Canule selon l'une des revendications 1 à 10 dont le tube (1) a un diamètre nominal de 1, 00 millimètre.

14. Canule selon la revendication 1 dont l'arête (3c) est plane et perpendiculaire à l'axe du tube.

15. Canule selon la revendication 14 dont les dites parois (3a ;3b) qui se rapprochent progressivement se terminent par deux parties de paroi (3a' ;3b') parallèles entre elles qui aboutissent à ladite arête.

16. Canule selon l'une des revendications 1 à 15 et dont le tube présente un oeil (5) qui aboutit sensiblement à la naissance de l'aplatissement du tube.

17. Canule selon l'une des revendications 1 à 16 et dont le tube présente un oeil (5) situé dans le prolongement de l'une des dites parois.

18. Canule selon l'une des revendications 1 à 17 et dont l'embase est munie d'un filtre (8)

## Claims

1. Canula for medical use for taking off or introducing a liquid through a stopper that is not slit in advance, formed by a hollow cylindrical metal tube (1) that has an open proximal end provided with a tubular connecting piece (2), which has a distal end (3) closed by an edge (3c) for perforating the stopper and which has between these ends at least one lateral eye (4; 4') communicating with the inside of the tube, **characterised in that** the distal end of the tube comprises a non-traumatic straight edge (3c) formed by two opposite tube walls (3a; 3b) that join progressively and are determined by flattening of the distal end of the tube.

2. Canula according to claim 1, the edge (3c) of which has a triangular-shaped cross-section.

3. Canula according to claim 2, the cross-section of the edge (3c) of which has an angle at the vertex of 60°, to within a tolerance.

4. Canula according to one of claims 1 to 3, the edge (3c) of which intersects the axis of the tube with an angle.

5. Canula according to claim 4, the edge (3c) of which forms a nominal angle of 75° with the axis of the tube.

6. Canula according to one of the preceding claims, the tube of which has an eye (4') situated at 90° from said opposite walls, to within a tolerance.

7. Canula according to one of claims 1 to 6, said opposite walls (3a; 3b) of which are curved towards each other.

8. Canula according to claim 7, said opposite walls (3a; 3b) of which are curved with a radius of curvature of 5.2 mm, to within a tolerance.

9. Canula according to one of claims 1 to 6, the tube of which has an eye (4; 4') the nominal distance of which to the end of said edge (3c) is no more than 2 millimetres.

10. Canula according to one of claims 1 to 7, the eye (4; 4') of which extends parallel to the axis of the canula over 1.2 millimetres, to within a tolerance.

11. Canula according to one of claims 1 to 8, the tube of which has an eye (4; 4') consisting of an opening formed in the wall of the tube and bordered by edges of this wall, said edges comprising two bevelled opposite edges (4a; 4c), respectively proximal and distal.

12. Canula according to claim 9, wherein said bevel of the bevelled edges forms an angle of 90°, to within a tolerance.

13. Canula according to one of claims 1 to 10, the tube (1) of which has a nominal diameter of 1.00 millimetre.

14. Canula according to claim 1, the edge (3c) of which is flat and perpendicular to the axis of the tube.

15. Canula according to claim 14, said walls (3a; 3b) of which, which progressively approach each other, terminate in two wall parts (3a'; 3b') parallel to each other, and which end at said edge.

16. Canula according to one of claims 1 to 15 and the tube of which has an eye (5) that ends substantially at the beginning of the flattening of the tube.

17. Canula according to one of claims 1 to 16, the tube of which has an eye (5) situated in line with one of said walls.

18. Canula according to one of claims 1 to 17, the base of which is provided with a filter (8).

## Patentansprüche

1. Kanüle für medizinischen Gebrauch zum Entnehmen oder Einbringen einer Flüssigkeit über einen nicht vorgeschlitzten Stopfen, gebildet durch ein hohles und zylindrisches metallisches Rohr (1), das ein offenes proximales Ende aufweist, welches mit einem röhrenförmigen unteren Anschlussteil (2) versehen ist, das ein distales Ende (3) aufweist, welches durch eine Kante (3c) geschlossen ist, um den Stopfen zu durchstechen, und das zwischen diesen Enden mindestens ein seitliches Auge (4; 4') aufweist, das mit dem Inneren des Rohrs kommuniziert, **dadurch gekennzeichnet, dass** das distale Ende des Rohrs eine gerade atraumatische Kante (3c) aufweist, die von zwei gegenüberliegenden Rohrwänden (3a; 3b) hervorgebracht wird, welche progressiv zusammenlaufen und durch Abflachung des distalen Endes des Rohrs gebildet sind.

2. Kanüle nach Anspruch 1, wobei die Kante (3c) einen Querschnitt mit dreieckiger Form aufweist.

3. Kanüle nach Anspruch 2, wobei der Querschnitt der Kante (3c) einen Winkel an der Spitze von 60°, innerhalb der Toleranzgrenze, aufweist.

4. Kanüle nach einem der Ansprüche 1 bis 3, wobei die Kante (3c) die Achse des Rohrs schräg schneidet.

5. Kanüle nach Anspruch 4, wobei die Kante (3c) mit der Achse des Rohrs einen nominalen Winkel von 75° bildet.

6. Kanüle nach einem der vorhergehenden Ansprüche, wobei das Rohr ein Auge (4') aufweist, das in einem Winkel von 90° zu den gegenüberliegenden Wänden, innerhalb der Toleranzgrenze, liegt.

7. Kanüle nach einem der Ansprüche 1 bis 6, wobei die gegenüberliegenden Wände (3a; 3b) zueinander gekrümmt sind.

8. Kanüle nach Anspruch 7, wobei die gegenüberliegenden Wände (3a; 3b) mit einem Krümmungsradius von 5,2 Millimetern, innerhalb der Toleranzgrenze, gekrümmt sind.

9. Kanüle nach einem der Ansprüche 1 bis 6, wobei das Rohr ein Auge (4; 4') aufweist, dessen nominaler Abstand zu dem Ende der Kante (3c) höchstens gleich 2 Millimeter beträgt.

10. Kanüle nach einem der Ansprüche 1 bis 7, wobei sich das Auge (4; 4') parallel zu der Achse der Kanüle über 1,2 Millimeter, innerhalb der Toleranzgrenze, erstreckt.

11. Kanüle nach einem der Ansprüche 1 bis 8, wobei das Rohr ein Auge (4; 4') aufweist, das von einer Öffnung gebildet ist, die in der Wand des Rohrs ausgeformt ist und von Abschnitten dieser Wand gesäumt ist, welche zwei gegenüberliegende abgeschrägte Abschnitte (4a; 4c), einen proximalen bzw. einen distalen, umfassen.

12. Kanüle nach Anspruch 9, wobei die Abschrägung der abgeschrägten Abschnitte einen Winkel von 90°, innerhalb der Toleranzgrenze, bildet.

13. Kanüle nach einem der Ansprüche 1 bis 10, wobei das Rohr (1) einen nominalen Durchmesser von 1,00 Millimeter hat.

14. Kanüle nach Anspruch 1, wobei die Kante (3c) eben und senkrecht zu der Achse des Rohrs ist.

15. Kanüle nach Anspruch 14, wobei die Wände (3a; 3b), die sich progressiv aneinander annähern, in zwei zueinander parallelen Wandteilen (3a'; 3b') enden, die bis zu der Kante reichen.

16. Kanüle nach einem der Ansprüche 1 bis 15, wobei das Rohr ein Auge (5) aufweist, das im Wesentlichen bis zum Ursprung der Abflachung des Rohrs reicht.

17. Kanüle nach einem der Ansprüche 1 bis 16, wobei das Rohr ein Auge (5) aufweist, das sich in der Verlängerung einer der beiden Wände befindet.

18. Kanüle nach einem der Ansprüche 1 bis 17, wobei das untere Anschlussteil mit einem Filter (8) versehen ist.
